(19) Europäisches Patentamt
European Patent Office
Office européen des brevets

(11) Numéro de publication : **0 172 761 B1**

(12) **FASCICULE DE BREVET EUROPÉEN**

(45) Date de publication du fascicule du brevet : **25.05.88**

(21) Numéro de dépôt : **85401423.0**

(22) Date de dépôt : **12.07.85**

(51) Int. Cl.⁴ : **C 07 C 43/317**, C 07 C 31/42, C 07 C 31/38, C 07 C 41/48, C 07 C 29/10, C 07 C 29/60, C 07 C 29/80

(54) **Procédé de préparation des hydrates de fluoral et d'hexafluoroacétone purs à partir d'hémiacétals.**

(30) Priorité : **18.07.84 FR 8411384**

(43) Date de publication de la demande : **26.02.86 Bulletin 86/09**

(45) Mention de la délivrance du brevet : **25.05.88 Bulletin 88/21**

(84) Etats contractants désignés : **AT BE CH DE FR GB IT LI NL SE**

(56) Documents cités :
**EP-A- 0 149 905**
**EP-A- 0 169 141**
**GB-A- 2 086 891**
**US-A- 2 681 370**

(73) Titulaire : **ATOCHEM**
**4 & 8, Cours Michelet La Défense 10**
**F-92800 Puteaux (FR)**

(72) Inventeur : **Cheminal, Bernard**
**Rés. Beauséjour St. Irénée 26, rue Soeur Bouvier**
**F-69005 Lyon (FR)**
Inventeur : **Mathais, Henri Hameau de Saint-Didier**
**Villa No. 2 Chemin de l'Indiennerie**
**F-69370 Saint-Didier-au-Mont-d'Or (FR)**
Inventeur : **Thomarat, Marc**
**22 boulevard de l'Europe**
**F-69310 Pierre Bénite (FR)**

(74) Mandataire : **Leboulenger, Jean et al**
**ATOCHEM Département Propriété Industrielle**
**F-92091 Paris la Défense 10 Cédex 42 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 172 761 B1

Jouve, 18, rue St-Denis, 75001 Paris, France

**Description**

La présente invention concerne la fabrication des hydrates de fluoral et d'hexafluoroacétone purs à partir d'hémiacétals. Ces hydrates sont utiles notamment comme intermédiaires pour la synthèse du trifluoro-2,2,2 éthanol et de l'alcool hexafluoro-1,1,1,3,3,3 isopropylique qui sont eux-mêmes des matières premières pour la synthèse d'anesthésiques.

Par hydrate de fluoral, on entend tous les composés suivants décrits dans la littérature.

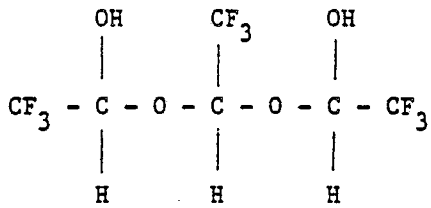

(équivalent à 3 $CF_3CHO.H_2O$)

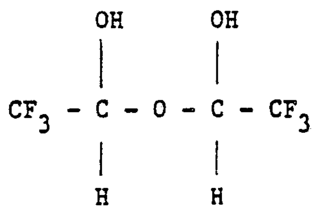

(équivalent à 2 $CF_3CHO.H_2O$)

(hémihydrate)

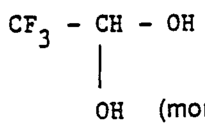

(équivalent à $CF_3CHO.H_2O$)

(monohydrate)

L'hydrate d'hexafluoroacétone a la structure d'un gem-diol :

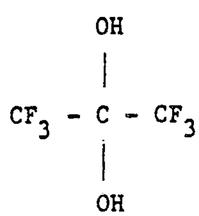

(voir W. J. MIDDLETON et D. W. WILEY, J.A.C.S., *86*, 4948 (1964), ainsi que C. G. KRESPAN et W. J. MIDDLETON, Fluorine Chemistry Reviews 1 (1), 145-196 (1967).

Ces hydrates à radicaux trifluorométhyle, de même que les hydrates à radicaux chlorodifluorométhyle ou dichlorofluorométhyle qui les accompagnent dans les produits bruts, ont des points de fusion de l'ordre de 50 à 60 °C et se subliment à leur point de fusion, ce qui rend impossible leur séparation par distillation (voir brevet FR-A-2 493 836).

Le brevet FR-A-2 493 836 décrit une technique de purification d'hexafluoroacétone contenant des chlorofluoroacétones ; elle consiste pour l'essentiel en une dégradation chimique à l'aide de composés basiques (hydroxyde de calcium) des sous-produits chlorés en composés séparables par décantation après addition d'un produit minéral (chlorure de calcium) qui provoque la démixion du mélange liquide ; on obtient alors un hydrate d'hexafluoroacétone pur. Ce procédé qui ne s'applique pas au fluoral nécessite un traitement approprié des eaux résiduaires. En outre, cette technique peut difficilement être conduite de façon continue.

Il est d'autre part connu (J. P. GUTHRIE, Can. J. Chem. *53*, 1975, 898-906) que, pour obtenir un hydrate d'aldéhyde à partir de l'hémiacétal correspondant, il est nécessaire d'employer une grande quantité d'eau (rapport molaire : eau/hémiacétal supérieur à 50).

L'invention a donc pour objet un procédé comme défini dans les revendications qui permet de remédier à ces inconvénients et fournit en continu ou en discontinu un hydrate pur, apte à une réaction ultérieure (par exemple, hydrogénolyse de l'hydrate de fluoral en trifluoro-2,2,2 éthanol).

Le procédé selon l'invention est caractérisé en ce que l'on fait réagir l'hémiacétal méthylique ou éthylique pur de formule :

$$\begin{array}{c} OH \\ | \\ CF_3 - C - OCH_3 \\ | \\ R \end{array} \text{(ou } C_2H_5\text{)} \qquad (I)$$

dans laquelle R est un atome d'hydrogène ou le radical trifluorométhyle, avec de l'eau dans une colonne à distiller, on distille en tête de colonne le méthanol ou l'éthanol formé, et on récupère en pied de colonne l'hydrate de fluoral (R = H) ou d'hexafluoroacétone (R = $CF_3$) pur, c'est-à-dire ne contenant pas d'hydrates chlorés.

Ce procédé qui correspond à l'équation réactionnelle suivante :

$$\begin{array}{c} OH \\ | \\ CF_3-C-OCH_3 \\ | \\ R \end{array} \text{(ou } OC_2H_5\text{)} + nH_2O \rightleftharpoons \begin{array}{c} CF_3-C{=}O.nH_2O \\ | \\ R \end{array} + \begin{array}{c} CH_3OH \nearrow \\ \text{ou } C_2H_5OH \end{array} \qquad (1)$$

va être maintenant décrit en détail dans le cas de l'hémiacétal méthylique du fluoral (R = H), mais s'applique facilement de la même façon et dans les mêmes conditions aux autres hémiacétals de formule (I).

L'hémiacétal méthylique utilisé dans le procédé selon l'invention est préparé à l'état brut par absorption dans le méthanol pur des gaz bruts de fluoruration catalytique du chloral, préalablement débarrassés de l'acide fluorhydrique et contenant essentiellement du fluoral ($CF_3CHO$), des sous-produits chlorés ($CF_2Cl$—$CHO$ et $CFCl_2CHO$), des composés légers ($CHF_3$, $CClF_3$, $CHF_2Cl$, $CHFCl_2$, CO) et de l'acide chlorhydrique. Cette absorption est de préférence réalisée à basse température (environ 0 °C) pour éviter la réaction secondaire :

$$CH_3OH + HCl \rightleftharpoons CH_3Cl + H_2O \qquad (2)$$

car la sous-production d'eau entraîne la formation des différents hydrates d'aldéhydes dont les caractéristiques physiques très proches (sublimation au point de fusion, points d'ébullition très voisins) ne permettent pas d'envisager une séparation d'avec l'hydrate de fluoral pur.

L'hémiacétal méthylique brut ainsi obtenu, éventuellement neutralisé par une solution aqueuse de carbonate de sodium, est ensuite purifié par distillation classique sous pression atmosphérique ou sous vide, mais de préférence en respectant un temps de séjour pas trop élevé (quelques minutes) dans le bouilleur de la colonne utilisée pour le distiller et, en l'absence de neutralisation préalable, en choisissant un appareil capable de résister à la corrosion par l'acide chlorhydrique anhydre gazeux.

Dans ces conditions, le taux de décomposition de l'hémiacétal $CF_3$—$CH(OH)$—$OCH$-3 est faible (inférieur à 1 %) et le rendement en produit pur (point d'ébullition = 97 °C/750 torr) est excellent (supérieur à 95 %). On peut prévoir si nécessaire une tour d'absorption au méthanol sur le circuit d'évent de la colonne de façon à récupérer les traces de fluoral décomposées. Après décomposition sous vide poussé à haute température, les produits lourds (composés chlorés) peuvent être recyclés dans l'étape précédente de fluoruration du chloral.

L'hémiacétal $CF_3$—$CH(OH)$—$OCH_3$ ainsi purifié peut être utilisé tel quel dans le procédé selon l'invention. Il est mis à réagir avec de l'eau dans un rapport molaire : $H_2O/CF_3$—$CH(OH)$—$OCH_3$ compris entre 1,5 et 3,0 (de préférence environ 2) à une température allant de 95 à 115 °C (de préférence, de 100 à 110 °C) sous pression atmosphérique, dans le bouilleur d'une colonne à distiller où le méthanol formé durant la réaction (1) est éliminé en tête de colonne au fur et à mesure de sa formation.

La colonne à distiller peut comporter un remplissage (anneaux, hélices, garnissages tissés) ou bien un ensemble de plateaux. Lorsqu'on opère en discontinu, l'eau et l'hémiacétal pur (soluble dans l'eau) sont introduits simultanément dans le bouilleur et la réaction menée à son terme, c'est-à-dire jusqu'à l'instant où les premières vapeurs d'hydrate atteignent la tête de la colonne et où cesse toute distillation du méthanol en tête. Pour la mise en œuvre continue du procédé selon l'invention, il est préférable de travailler dans une colonne à plateaux qui assurent sur chaque plateau le meilleur contact possible entre l'eau (introduite sur l'un des plateaux au-dessus du bouilleur) et l'hémiacétal pur (introduit dans le bouilleur ou, de préférence, entre le bouilleur et le plateau d'introduction de l'eau).

Le méthanol produit dans le procédé selon l'invention est facilement recyclable à l'étape précédente d'absorption du fluoral gazeux brut décrit ci-dessus.

Le procédé selon l'invention résout plus particulièrement le problème de la séparation du trifluoro-2,2,2 éthanol et du méthanol qui forment entre eux un azéotrope à maximum ce qui provoque une perte de productivité en trifluoro-2,2,2 éthanol préjudiciable à la rentabilité de sa fabrication industrielle et à la pureté du méthanol recyclé.

Les exemples suivants illustrent l'invention, sans la limiter.

Exemple 1 (Préparation de l'hydrate de fluoral pur)

On utilise une colonne à distiller d'une longueur de 800 mm et d'un diamètre intérieur de 25 mm remplie de tampons métalliques MULTIKNIT. Le bouilleur est chauffé au moyen d'un bain d'huile (thermostaté) et le condenseur est alimenté avec un mélange réfrigérant aux alentours de + 3 à + 5 °C. L'appareil fonctionne sous pression atmosphérique ; un répartiteur automatique de reflux (TIMER) règle la proportion de liquide prélevé en tête de colonne.

On introduit dans le bouilleur 3,42 moles d'eau et 3,26 moles d'hémiacétal $CF_3—CH(OH)—OCH_3$ pur, obtenu comme indiqué précédemment. Le tableau 1 suivant résume les conditions de marche et indique le volume de méthanol distillé.

Tableau 1

| Durée (heures et minutes) | Température °C Tête | Pied | Taux de reflux | Volume de méthanol distillé (en ml) | Observations |
|---|---|---|---|---|---|
| 0 H 15 | - | 92/93 | - | - | Début de l'ébullition |
| 1 H 00 | 63,8 | 96 | - | - | |
| 1 H 30 | 63,9 | 98 | 9/1 | 10 | |
| 2 H 00 | 64,0 | 100 | " | 26 | |
| 2 H 30 | 63,9 | 102 | " | 44 | |
| 3 H 00 | 64,0 | 103 | " | 57 | |
| 3 H 30 | 87,0 | 104 | " | 71 | Reflux plus difficile |

On rajoute alors 3,42 moles d'eau et la distillation en tête redémarre (voir tableau 2 ci-dessous). Après cette seconde addition d'eau, le rapport molaire : eau/hémiacétal est égal à 2,1.

Tableau 2

| Durée (heures et minutes) | Température °C Tête | Pied | Taux de reflux | Volume de méthanol distillé (en ml) | Observations |
|---|---|---|---|---|---|
| 0 H | - | - | - | - | Addition d'eau |
| 0 H 15 | 64,2 | 102 | - | - | Début du soutirage |
| 1 H 00 | 64,0 | 105,2 | 20/1 | 11 | |
| 1 H 30 | 64,0 | 107,0 | " | 19 | |
| 3 H 00 | 64,0 | 108,5 | " | 35 | |
| 4 H 45 | 64,0 | 109,2 | " | 35 | |
| 6 H 15 | 70,0 | 110,0 | " | 50 | Fin de l'opération |

Le taux de récupération du méthanol distillé par rapport à la quantité théorique (104 g) contenu dans l'hémiacétal de départ correspond à 93 % ; la retenue de colonne qui contient encore du méthanol lorsque la température de tête atteint puis dépasse 70 °C, correspond aux 7 % qui manquent au bilan. Une analyse du méthanol de tête indique la présence de 0,2 % en poids d'eau (qui peut être éliminée aisément sur tamis moléculaire). Les impuretés organiques qui l'accompagnent (moins de 0,01 % en poids) ne gênent pas et ce méthanol peut donc être recyclé sans aucun inconvénient à l'absorption du fluoral gazeux brut ; en cas d'accumulation rédhibitoire de ces impuretés, on peut aisément les séparer du méthanol par distillation en discontinu.

En pied de colonne, on récupère un produit dont l'analyse R.M.N. montre qu'il contient moins de 0,1 % en poids de méthanol. Il est constitué pour environ 95 % en poids par le monohydrate $CF_3—CH(OH)_2$, caractérisé par son analyse R.M.N. du fluor $F^{19}$ et du proton, et pour environ 5 % par l'hémihydrate $CF_3—CH(OH)—O—CH(OH)—CF_3$. L'eau restante est associée au monohydrate.

Exemple 2 (Hydrogénolyse de l'hydrate de fluoral)

Cet exemple illustre l'emploi de l'hydrate de fluoral (liquide) préparé à l'exemple 1 pour la fabrication du trifluoro-2,2,2 éthanol.

Dans un autoclave ayant un volume utile d'environ 100 ml et muni d'une agitation par barreau magnétique, on introduit 0,50 g d'un catalyseur à 5 % en poids de palladium déposé sur charbon actif (commercialisé par la société ENGELHARDT) et 20,7 g (0,207 mole) de trifluoro-2,2,2 éthanol pur. Sous une pression d'hydrogène de 45 bars, on chauffe ce mélange à 120 °C pendant une demi-heure, puis on introduit progressivement (en 68 minutes) 47,1 g du produit recueilli en pied de colonne à l'exemple 1 et 0,6 g de triéthylamine pure (co-catalyseur), tout en maintenant le mélange à 120 °C et sous 45 bars par additions successives d'hydrogène frais.

A la fin de l'introduction de l'hydrate de fluoral, l'absorption d'hydrogène dure encore 5 minutes environ puis cesse. La vitesse de réaction pendant l'injection du réactif représente 99 % de la vitesse de réaction globale.

L'autoclave est refroidi rapidement et le catalyseur est séparé par filtration à l'extérieur du réacteur, lavé 8 fois avec de l'eau et enfin séché sous vide, puis recyclé pour deux autres opérations identiques (même substrat et même charge catalytique).

L'analyse chromatographique du mélange obtenu indique une conversion totale de l'hydrate de fluoral et un rendemant quantitatif en trifluoro-2,2,2 éthanol. Ce produit brut est facilement purifié par les méthodes conventionnelles (distillation et éventuellement séchage final sur tamis moléculaire pour séparer l'eau).

Dans le tableau 3 ci-après, figurent les résultats obtenus au cours de ces trois opérations consécutives sur la même charge de catalyseur.

Tableau 3

| Opération N° | Durée de la réaction (minutes) | Transformation pendant l'introduction % | PH | Vitesse moyenne (moles de $CF_3CH_2OH$ par at-g. de Pd) |
|---|---|---|---|---|
| 1 | 73 | 99,0 | 9,95 | 983 |
| 2 | 52 | 98,5 | 9,85 | 1458 |
| 3 | 52 | 98,6 | 9,75 | 1484 |

L'activité catalytique n'est pas affectée par l'utilisation de l'hydrate de fluoral obtenu par le procédé selon l'invention et dont l'emploi présente l'avantage de ne nécessiter pour l'obtention de trifluoro-2,2,2 éthanol rigoureusement pur qu'une distillation sommaire de la solution aqueuse de cet alcool. On évite aussi de cette manière la sous-production de produits extrêmement toxiques ($CFH_2—CH_2OH$) qui peuvent accompagner le trifluoro-2,2,2 éthanol.

**Revendications**

1. Procédé de préparation des hydrates de fluoral et d'hexafluoroacétone purs, caractérisé en ce que l'on fait réagir dans une colonne à distiller sous pression atmosphérique à une température comprise entre 95 et 115 °C l'hémiacétal méthylique ou éthylique pur de formule :

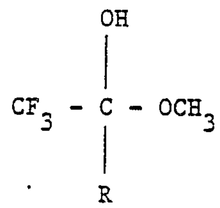 (ou $OC_2H_5$)

dans laquelle R est un atome d'hydrogène ou le radical trifluorométhyle, avec de l'eau dans un rapport molaire : eau/hémiacétal allant de 1,5 à 3, on distille en tête de colonne le méthanol ou l'éthanol au fur et à mesure de sa formation et on récupère en pied de colonne l'hydrate de fluoral (R = H) ou d'hexafluoroacétone ($R = CF_3$) pur.

2. Procédé selon la revendication 1, dans lequel on opère en discontinu en introduisant l'hémiacétal et l'eau dans le bouilleur de la colonne à distiller.

3. Procédé selon la revendication 1, dans lequel on opère en continu dans une colonne à plateaux en introduisant l'eau sur l'un des plateaux au-dessus du bouilleur et l'hémiacétal entre le bouilleur et le plateau d'introduction de l'eau.

4. Procédé selon l'une des revendications 1 à 3, dans lequel l'eau et l'hémiacétal sont introduits dans un rapport molaire : eau/hémiacétal de 2 environ.

5. Procédé selon l'une des revendications 1 à 4, dans lequel on opère à une température comprise entre 100 et 110 °C.

6. Procédé selon l'une des revendications 1 à 5, dans lequel on utilise un hémiacétal obtenu par absorption à basse température dans le méthanol pur ou l'éthanol pur absolu ou azéotropique, des gaz provenant de la fluoruration catalytique du chloral ou de l'hexachloroacétone, préalablement débarrassés de l'acide fluorhydrique, puis distillation sous pression atmosphérique ou sous vide.

7. Procédé selon l'une des revendications 1 à 6, dans lequel le méthanol ou l'éthanol obtenu en tête de la colonne à distiller est utilisé pour l'absorption des gaz de fluoruration.

8. Procédé de fabrication de trifluoro-2,2,2 éthanol ou d'alcool hexafluoro-1,1,1,3,3,3 isopropylique, caractérisé en ce que l'on prépare les hydrates de fluoral ou d'hexafluoroacétone par un procédé selon l'une des revendications 1 à 7 et en ce que l'on soumet les produits ainsi obtenus à l'hydrogénolyse catalytique en phase liquide.

**Claims**

1. Process for the preparation of pure hydrates of fluoral and of hexafluoroacetone, characterized in that pure methyl or ethyl hemiacetal of formula :

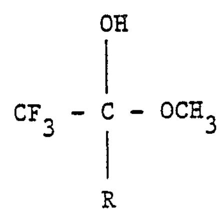 (or $OC_2H_5$)

in which R is a hydrogen atom or the trifluoromethyl radical, is reacted, under atmospheric pressure and at a temperature of between 95 and 115 °C, with water in a distillation column with a molar ratio : water/hemiacetal ranging from 1.5 to 3, the methanol or ethanol is distilled at the top of the column as it is formed and the pure hydrate of fluoral (R = H) or of hexafluoroacetone ($R = CF_3$) is recovered at the bottom of the column.

2. Process according to Claim 1, in which the operation is carried out noncontinuously by introducing hemiacetal and water into the boiler of the distillation column.

3. Process according to Claim 1, in which the operation is carried out continuously in a plate column by introducing water on one of the plates above the boiler and hemiacetal between the boiler and the plate on which water is introduced.

4. Process according to one of Claims 1 to 3, in which water and hemiacetal are introduced in a molar ratio : water/hemiacetal of approximately 2.

5. Process according to one of Claims 1 to 4, in which the operation in carried out at a temperature between 100 and 110 °C.

6. Process according to one of Claims 1 to 5, in which use is made of a hemiacetal obtained by absorbing at a low temperature, in pure methanol or pure ethanol, absolute or azeotropic, gases originating from the catalytic fluorination of chloral or of hexachloroacetone, which have been freed from hydrofluoric acid beforehand, followed by distillation at atmospheric pressure or under vacuum.

7. Process according to one of Claims 1 to 6, in which the methanol or ethanol obtained at the top of the distillation column is used to absorb the gases from fluorination.

8. Process for the manufacture of 2,2,2-trifluoroethanol or 1,1,1,3,3,3-hexa-fluoroisopropyl alcohol, characterized in that the hydrates of fluoral or of hexafluoroacetone are prepared by a process according to one of Claims 1 to 7, and in that the products thus obtained are subjected to catalytic hydrogenolysis in liquid phase.

**Patentansprüche**

1. Verfahren zur Herstellung von reinen Hydraten von Trifluoracetaldehyd oder Hexafluoraceton, dadurch gekennzeichnet, daß man in einer Destillationskolonne bei Atmosphärendruck und bei einer Temperatur zwischen 95 und 115 °C das reine Methyl- oder Ethylhemiacetal der Formel :

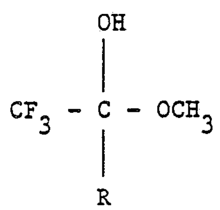

(oder $OC_2H_5$)

in der R ein Wasserstoffatom oder der Rest Trifluormethyl ist, mit Wasser in einem molaren Verhältnis : Wasser/Hemiacetal zwischen 1,5 und 3 reagieren läßt, daß man am Kopf der Kolonne das Methanol oder das Ethanol nach und nach bei seiner Bildung abdestilliert und daß man am Fuß der Kolonne das reine Hydrat des Trifluoracetaldehyds (R = H) oder des Hexafluoracetons (R = $CF_3$) gewinnt.

2. Verfahren nach Anspruch 1, bei dem man diskontinuierlich unter Einführung des Hemiacetals und des Wassers in das Siedegefäß der Destillationskolonne arbeitet.

3. Verfahren nach Anspruch 1, bei dem man kontinuierlich in einer Kolonne mit Böden arbeitet, wobei das Wasser auf einem der Böden oberhalb des Siedegefäßes eingebracht wird und das Hemiacetal eingebracht wird zwischen dem Siedegefäß und dem Bodennn auf dem das Wasser eingebracht wird.

4. Verfahren nach einem der Ansprüche 1 bis 3, bei dem Wasser und Hemiacetal in einem molaren Verhältnis Wasser/Hemiacetal von ungefähr 2 eingebracht werden.

5. Verfahren nach einem der Ansprüche 1 bis 4, bei dem man bei einer Temperatur zwischen 100 und 110 °C arbeitet.

6. Verfahren nach einem der Ansprüche 1 bis 5, bei dem man ein Hemiacetal verwendet, das surch Absorption von bei der katalytischen Fluorierung des Chlorals oder des Hexachloracetons erhaltenen Gasen, die zuvor von Fluorwasserstoffsäure befreit wurden, bei tiefer Temperatur in reinem Methanol oder reinem absoluten oder azeotropen Ethanol erhalten wurde und anschließende Destillation bei Atmosphärendruck oder im Vakuum.

7. Verfahren nach einem der Ansprüche 1 bis 6, bei dem sas Methanol oder Ethanol, das am Kopf der Destillationskolonne erhalten wird, zur Absorption der Gase aus der Fluorierung verwendet wird.

8. Verfahren zur Herstellung von 2,2,2-Trifluorethanol oder 1,1,1,3,3,3-Hexafluorisopropylalkohol, dadurch gekennzeichnet, daß man die Hydrate des Trifluoracetaldehyds oder des Hexafluoracetons in einem Verfahren gemäß einem der Ansprüche 1 bis 7 herstellt und die so erhaltenen Produkte der katalytischen Hydrogenolyse in der Flüssigphase unterwirft.